(19) Europäisches Patentamt
European Patent Office
Office européen des brevets

(11) Veröffentlichungsnummer: **0 007 450**
**A1**

(12) # EUROPÄISCHE PATENTANMELDUNG

(21) Anmeldenummer: 79102146.2

(22) Anmeldetag: 28.06.79

(51) Int. Cl.[3]: C 07 D 495/04
C 07 D 491/04, A 61 K 31/335
A 61 K 31/38, A 61 K 31/40
A 61 K 31/55
//(C07D491/04, 313/00, 209/00),
(C07D495/04, 337/00, 209/00)

(30) Priorität: 07.07.78 CH 7420/78

(43) Veröffentlichungstag der Anmeldung:
06.02.80 Patentblatt 80/3

(84) Benannte Vertragsstaaten:
AT BE CH DE FR GB IT LU NL SE

(71) Anmelder: CIBA-GEIGY AG
Patentabteilung Postfach
CH-4002 Basel(CH)

(72) Erfinder: Blattner, Hans
Burgstrasse 116
CH-4125 Riehen(CH)

(72) Erfinder: Storni, Angelo, Dr.
Im Feuerbusch 3
CH-4310 Rheinfelden(CH)

(54) **Azatetracyclische Carbonitrile, ihre Herstellung, pharmazeutische Präparate, die diese enthalten und deren Verwendung.**

(57) Azatetracyclische Carbonitrile der Formel I

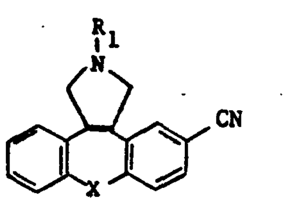

(I)

in welcher $R_1$ Wasserstoff, Niederalkyl, Cycloalkylniederalkyl, Niederalkenyl, freies, veräthertes oder verestertes Hydroxyniederalkyl ist, und X Epoxy, Epithio, Methylen, oder einen zweiwertigen Rest der Teilformel

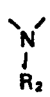

(Ia)

bedeutet, in der $R_2$ Wasserstoff oder Niederalkyl darstellt, und Salze von solchen Verbindungen, ihre Herstellung, pharmazeutische Präparate, die diese enthalten und deren Verwendung.

EP 0 007 450 A1

CIBA-GEIGY AG 4-11908/+

Basel (Schweiz)

Azatetracyclische Carbonitrile, ihre Herstellung, pharmazeutische Präparate, die diese enthalten und deren Verwendung

Die Erfindung betrifft neue azatetracyclische Carbonitrile, insbesondere 2-substituierte 5-Cyano-2,3-dihydro-1H-dibenzo[2,3:6,7]thiepino[4,5-c]pyrrole und entsprechende Oxepino-, Azepino- und Cycloheptatrienoverbindungen und Salze von solchen Verbindungen, Verfahren zu ihrer Herstellung, Arzneimittel, welche die neuen Verbindungen enthalten, und ihre Verwendung.

Die erfindungsgemässen azatetracyclischen Carbonitrile entsprechen der Formel I

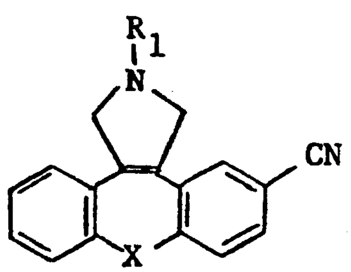

(I)

in welcher $R_1$ Wasserstoff, Niederalkyl, Cycloalkylniederalkyl, Niederalkenyl, freies, veräthertes oder verestertes Hydroxyniederalkyl ist, und X Epoxy, Epithio, Methylen, oder einen zweiwertigen Rest der Teilformel

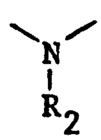

(Ia)

bedeutet, in der $R_2$ Wasserstoff oder Niederalkyl darstellt.

$R_1$ enthält als Niederalkyl vorzugsweise 1 bis 7 Kohlenstoffatome. Diese Niederalkylgruppen, die geradkettig oder verzweigt sein können, sind z.B. Methyl, Aethyl, Propyl, Isopropyl, Butyl, Isobutyl oder Tertiärbutyl.

Als Cycloalkylniederalkyl enthält der Rest $R_1$ vorzugsweise 4 bis 8 Kohlenstoffatome. Cycloalkylniederalkyl ist also z.B. Cyclopropylmethyl, Cyclobutylmethyl und insbesondere Cyclopentylmethyl, Cyclohexylmethyl, weiter z.B. Cyclopropyläthyl, Cyclobutyläthyl, Cyclopentyläthyl, Cyclohexyläthyl.

Als Niederalkenyl enthält der Rest $R_1$ vorzugsweise 3 bis 4 Kohlenstoffatome und insbesondere 3 Kohlenstoffatome. Niederalkenyl liegt z.B. als Allyl oder 2-Methylallyl vor.

Substituenten von Niederalkyl sind freies, veräthertes oder verestertes Hydroxy. Solche Reste sind z.B. Hydroxyniederalkyl, Niederalkoxyniederalkyl oder Alkanoyloxyniederalkyl.

Im Hydroxyniederalkyl als Rest $R_1$ ist die Hydroxylgruppe durch mindestens 2 Kohlenstoffatome vom Ringstickstoffatom getrennt. Dieser Rest enthält 2 bis 8 und bevorzugt 2 bis 4 Kohlenstoffatome. Hydroxyniederalkyl, das geradkettig oder verzweigt sein kann, ist z.B. das 1-Methyl-2-hydroxyäthyl, 2-Hydroxypropyl, 1- oder 2-Methyl-2-hydroxy-propyl, und insbesondere das 2-Hydroxy-äthyl und das 3-Hydroxypropyl.

Im Niederalkoxyniederalkyl als Rest $R_1$ ist das Sauerstoffatom durch mindestens 2 Kohlenstoffatome vom Ringstickstoffatom getrennt. Dieser Rest enthält z.B. 3 bis 10 und bevorzugt 3 bis 6 Kohlenstoffatome. Dieses Niederalkoxyniederalkyl ist z.B. das 2-Methoxypropyl, 2- oder 3-Aethoxypropyl, 2- oder 3-Propoxy-propyl, 3-Isopropoxypropyl und insbesondere das 2-Methoxy- oder 2-Aethoxy-äthyl und vor allem das 3-Methoxy-propyl.

Im Alkanoyloxyniederalkyl als Rest $R_1$ ist das Sauerstoffatom durch mindestens 2 Kohlenstoffatome vom Ringstickstoffatom getrennt. Dieser Rest enthält z.B. 3 bis 21 und bevorzugt 4 bis 11 Kohlenstoffatome. Dieser Rest ist z.B. 2-Formyloxy-äthyl, 2-Acetyloxy-äthyl, 2-Propionyloxy-äthyl, 2-Acetyloxy-propyl, 2-Methyl-3-acetyloxy-propyl oder 2- oder 3-Propionyloxy-propyl und insbesondere 3-Acetyloxypropyl und 3-Oktanoyloxypropyl.

$R_2$ ist vorzugsweise Wasserstoff oder Niederalkyl mit höchstens 4 Kohlenstoffatomen, wie Propyl, Butyl, Isobutyl und vor allem Methyl oder Aethyl.

Salze von Verbindungen der Formel I sind Säureadditionssalze, insbesondere pharmazeutisch annehmbare Säureadditionssalze, z.B. mit anorganischen Säuren, wie Chlorwasserstoffsäure, Bromwasserstoffsäure, Schwefelsäure oder Phosphorsäure, oder mit organischen Säuren, wie organischen Carbon- und Sulfonsäuren, wie Methansulfonsäure, Aethansulfonsäure, 2-Hydroxyäthansulfonsäure, Essigsäure, Aepfelsäure, Weinsäure, Zitronensäure, Milchsäure, Oxalsäure, Bernsteinsäure, Fumarsäure, Maleinsäure, Benzoesäure, Salicylsäure, Phenylessigsäure, Mandelsäure oder Embonsäure.

Die neuen azatetracyclischen Carbonitrile der allgemeinen Formel I und ihre Säureadditionssalze weisen wertvolle pharmakologische, z.B. auf das Zentralnervensystem wirkende, Eigenschaften auf. Sie zeichnen sich in erster Linie durch langandauernde zentraldämpfende, erregungshemmende (Amphetamin-antagonistische) Wirkungen aus, was anhand von pharmakologischen Versuchen nachgewiesen werden kann. So zeigen sie an der Ratte im Amphetamin-Antagonismus-Test (Nemegeers und Janssen, Arzneimittelforsch., Bd. 24, S. 45 (1974)) in einem Dosenbereich von 0,01 bis 1,0 mg/kg i.p. oder per os eine erregungshemmende Wirkung. Die neuen azatetracyclischen Carbonitrile der allgemeinen Formel I und ihre pharmazeutisch annehmbaren Säureadditionssalze können deshalb als tranquillisierende, antipsychotische und erregungshemmende Verbindungen zur Behandlung von Erregungszuständen verwendet werden.

Die Erfindung betrifft in erster Linie Verbindungen der Formel I, worin $R_1$ Wasserstoff, Niederalkyl, z.B. Methyl, Aethyl, Isopropyl und n-Butyl, Cycloalkylniederalkyl mit 4-8 Kohlenstoffatomen, z.B. Cyclopentylmethyl und Cyclohexylmethyl, Niederalkenyl, z.B. Allyl, Hydroxyniederalkyl, z.B. 2-Hydroxyäthyl und 3-Hydroxy-propyl, Niederalkoxyniederalkyl, z.B. 3-Methoxy- und 3-Aethoxypropyl, oder Alkanoyloxy-niederalkyl, z.B. 3-Acetyloxy-propyl und 3-Oktanoyloxy-propyl, bedeutet, X Epoxy, Epithio, Methylen oder den zweiwertigen Rest der Teilformel Ia bedeutet,

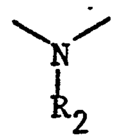

(Ia)

in welcher $R_2$ für Wasserstoff oder Niederalkyl mit bis 4 Kohlenstoffatomen, vorzugsweise Methyl oder Aethyl steht, und Salze, insbesondere Säureadditionssalze in erster Linie pharmazeutisch annehmbare Säureadditionssalze davon.

Die Erfindung betrifft insbesondere Verbindungen der Formel I, worin $R_1$ Wasserstoff, Niederalkyl, z.B. Methyl, Aethyl oder Propyl, Cycloalkylniederalkyl mit 4-8 Kohlenstoffatomen, z.B. Cyclopentylmethyl und Cyclohexylmethyl, Niederalkenyl, z.B. Allyl oder Hydroxyniederalkyl, z.B. 2-Hydroxyäthyl und 3-Hydroxypropyl, X Epoxy oder Epithio, darstellt, und Salze, insbesondere Säureadditionssalze, in erster Linie pharmazeutisch annehmbare Säureadditionssalze davon.

Die Erfindung betrifft vor allem Verbindungen der Formel I, worin $R_1$ Wasserstoff, Methyl, Aethyl, Propyl, Cyclopentylmethyl oder 3-Hydroxypropyl, und X Epoxy oder Epithio bedeutet, wie das 2-Methyl-5-cyano-2,3-dihydro-1H-dibenzo[2,3:6,7]thiepino[4,5-c]pyrrol, 2-Aethyl-5-cyano-2,3-dihydro-1H-dibenzo[2,3:6,7]thiepino[4,5-c]pyrrol, 2-(Cyclopentylmethyl)-5-cyano-2,3-dihydro-1H-dibenzo[2,3:6,7]thiepino[4,5-c]pyrrol, 2-Propyl-5-cyano-2,3-dihydro-1H-dibenzo[2,3:6,7]thiepino[4,5-c]pyrrol, 5-Cyano-2,3-dihydro-1H-dibenzo[2,3:6,7]thiepino[4,5-c]pyrrol-3-propanol oder 2-Aethyl-5-cyano-2,3-dihydro-1H-dibenz[2,3:6,7]oxepino[4,5-c]pyrrol, und Salze davon, insbesondere Säureadditionssalze, in erster Linie pharmazeutisch annehmbare Säureadditionssalze davon.

Die Verbindungen der Formel I werden in an sich bekannter Weise hergestellt. So erhält man sie z.B., indem man einen reaktionsfähigen Diester eines Dimethanols der Formel

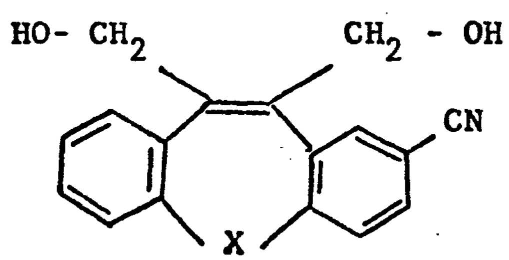

(II)

mit einer Verbindung der Formel

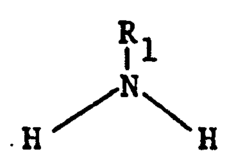

(III)

umsetzt.

Als reaktionsfähige Diester eines Dimethanols der Formel II können Ester mit starken anorganischen Säuren, wie z.B. Chlorwasserstoff-, Jodwasserstoffsäuren oder insbesondere Bromwasserstoffsäure eingesetzt werden. Ferner können auch entsprechende Diester von starken organischen Säuren, z.B. von Sulfonsäuren, wie Methansulfonsäure, Benzolsulfonsäure, p-Chlor- oder p-Brom-benzolsulfonsäure oder p-Toluolsulfonsäure, verwendet werden. Diese Diester der Verbindungen der Formel II werden vorzugsweise in einem geeigneten inerten Lösungsmittel bei einer Reaktionstemperatur von 20 bis 130°C umgesetzt. Als inerte Lösungsmittel eignen sich beispielsweise Kohlenwasserstoffe, wie Benzol oder Toluol, Halogenkohlenwasserstoffe, wie Chloroform, niedere Alkohole, wie Aethanol und insbesondere Methanol, ätherartige Flüssigkeiten, wie Aether oder Dioxan, sowie niedere Alkanone, z.B. Aceton, Methyläthylketon oder Diäthylketon oder Gemische von solchen Lösungsmitteln, z.B. Benzol-Methanol.

Bei der Umsetzung von einem Moläquivalent Diester eines Dimethanols der Formel II mit einem Moläquivalent freier Base der Formel III werden zwei Moläquivalente Säure abgespalten, die vorzugsweise an ein säurebindendes Mittel gebunden werden. Als säurebindende Mittel eignen sich z.B. Alkalicarbonate, wie Kaliumcarbonat, oder z.B. Alkalihydroxide, wie Natriumhydroxid oder Kaliumhydroxid, oder überschüssige Base der Formel III, ferner tertiäre organische Basen, wie Pyridin und insbesondere Triäthylamin oder N-Aethyl-diisopropylamin.

Die unmittelbaren Ausgangsstoffe, also die reakttionsfähigen Diester von Dimethanolen der Formel II können in an sich bekannter Weise, z.B. aus den entsprechenden Dimethylverbindungen durch Bromieren, z.B. mit N-Bromsuccinimid, hergestellt werden.

Nach einem weiteren Verfahren erhält man die Verbindungen der Formel I, indem man eine Verbindung der Formel

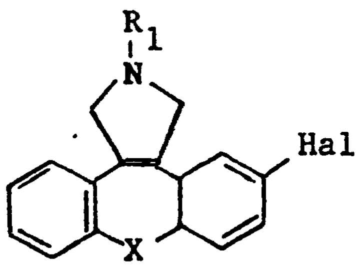

(IV)

in welcher Hal ein Halogenatom darstellt, mit einer Cyanidverbindung umsetzt.

Als Reste Hal in Verbindungen der Formel IV wählt man Chlor oder Jod, aber vorzugsweise Brom. Cyanidverbindungen sind in erster Linie Alkalimetall- oder Schwermetallcyanide. Als Alkalicyanid wird Natriumcyanid bevorzugt. Aber ganz besonders eignet sich Kupfer-I-cyanid, als Vertreter der Schwermetallcyanide. Die Reaktion kann in An- oder Abwesenheit von Lösungsmitteln durchgeführt werden, und dies in einem Termperaturbereich von 80° bis 250°. Als Lösungsmittel eignen sich besonders Pyridin, Chinolin, Dimethylformamid, 1-Methyl-2-pyrrolidinon und Hexamethylphosphorsäuretriamid. Die beiden letztgenannten eignen sich besonders für Kupfer-I-cyanid als Cyanierungsmittel.

Die Ausgangsstoffe der Formel IV sind bekannt oder können nach an sich bekannten Methoden, wie z.B. analog dem erstgenannten Verfahren, hergestellt werden. In diesem Zusammenhang wird u.a. auf DOS 1 959 405,

1 965 969 und 2 125 634 verwiesen.

Gegebenenfalls kann eine Verbindung der Formel I, deren Rest $R_1$ Wasserstoff darstellt, in ein Verfahrensprodukt übergeführt werden, dessen Rest $R_1$ eine der anderen Bedeutungen hat.

So kann z.B. eine N-Substitution erfolgen, entweder durch Behandeln mit einem reaktionsfähigen Ester eines entsprechenden Alkohols der Formel $R_1'$ - OH, worin $R_1'$ die gleiche Bedeutung wie $R_1$ in Formel I hat mit Ausnahme von Wasserstoff, oder durch eine Umsetzung mit einem entsprechenden Aldehyd oder Keton unter reduzierenden Bedingungen.

Die Umsetzung von Verbindungen der Formel I, in der $R_1$ Wasserstoff bedeutet, mit einem reaktionsfähigen Ester einer Hydroxyverbindung der Formel $R_1'$-OH wird vorzugsweise in einem Lösungsmittel bei einer Reaktionstemperatur von 20 bis 130°C, insbesondere bei der Siedetemperatur des Lösungsmittels, und gegebenenfalls in Gegenwart eines säurebindenden Mittels vorgenommen.

Als reaktionsfähige Ester können z.B. Halogenide, wie Chloride oder Bromide, ferner Sulfonsäureester, wie der p-Toluolsulfonsäureester oder Schwefelsäureester verwendet werden. Als säurebindende Mittel eignen sich Alkalimetallcarbonate, wie z.B. Kaliumcarbonat, oder Alkalihydroxide, wie z.B. Natriumhydroxid, oder tertiäre organische Basen, wie z.B. Pyridin oder das N-Aethyldiisopropylamin. Geeignete Lösungsmittel sind solche Lösungsmittel, die unter den Reaktionsbedingungen inert sind, beispielsweise Kohlenwasserstoffe, wie Benzol oder Toluol, ferner Alkanole, wie z.B. Methanol oder Aethanol, oder Alkanone, wie Aceton oder Methyläthylketon.

Aldehyde und Ketone, die den Alkoholen der Formel $R_1'$ - OH entsprechen, sind z.B. niedere aliphatische Aldehyde oder Ketone sowie niedere freie, veresterte oder verätherte Hydroxyoxoalkane. Das erhaltene Reaktionsprodukt bei der Umsetzung dieser Aldehyde oder Ketone mit den genannten Verbindungen der Formel I kann im gleichen Arbeitsgang oder anschliessend reduziert werden.

Die Aldehyde, z.B. Formaldehyd oder Acetaldehyd, oder die Ketone, z.B. Aceton, werden beispielsweise mit den genannten Verbindungen der Formel I in einem inerten Lösungsmittel auf ca. 30° bis 100°C erwärmt, und gleichzeitig oder anschliessend wird das Reaktionsgemisch mit Wasserstoff in Gegenwart eines Katalysators hydriert. Geeignete Lösungsmittel sind z.B. Alkanole, wie Methanol oder Aethanol, und geeignete Katalysatoren Edelmetallkatalysatoren, wie Palladium auf Kohle.

Anstelle von Wasserstoff in Gegenwart eines Katalysators können aber auch andere Reduktionsmittel, z.B. Ameisensäure, für die reduktive Alkylierung verwendet werden. Nach dieser Variante des Verfahrens werden die genannten Verbindungen der Formel I mit Ameisensäure und den genannten Typen von Aldehyden oder Ketonen, insbesondere Formaldehyd, vorzugsweise im Ueberschuss ohne andere Lösungsmittel, erwärmt.

Ferner kann gegebenenfalls eine Verbindung der Formel I, dessen Rest $R_1$ eine Hydroxyniederalkylgruppe darstellt, zu einer Verbindung acyliert werden, deren Rest $R_1$ eine veresterte Hydroxyniederalkylgruppe bedeutet.

Die Acylierung kann z.B. mit einem Carbonsäureanhydrid, inkl. einem entsprechenden Carbonsäurehalogenid, bei einer Reaktionstemperatur zwischen ca. 20 und 100°C vorgenommen werden. Da die Kondensation unter Abspaltung von Säure vor sich geht, ist es zweckmässig, dem Reaktionsgemisch ein säurebindendes Mittel, z.B. eine tertiäre organische Base, wie Pyridin, beizufügen. Ueberschüssige tertiäre organische Base kann auch als Lösungsmittel verwendet werden. Ferner kann man als Lösungsmittel Kohlenwasserstoffe, z.B. Benzol oder Toluol, oder Halogenkohlenwasserstoffe, z.B. Chloroform, einsetzen.

Ferner wird gegebenenfalls ein erhaltenes Verfahrensprodukt der allgemeinen Formel I, dessen Rest $R_1$ eine freie Hydroxyniederalkylgruppe darstellt, zu einem Reaktionsprodukt veräthert, dessen Rest $R_1$ eine verätherte niedere Hydroxyalkylgruppe bedeutet.

Die Verätherung kann vorgenommen werden, indem man die Hydroxyalkylverbindung, vorzugsweise in einem Lösungsmittel, in Gegenwart eines Kondensationsmittels mit einem reaktionsfähigen Ester eines Alkohols umsetzt. Als reaktionsfähige Ester eignen sich beispielsweise Ester der Halogenwasserstoffsäuren, z.B. der Bromwasserstoff- oder Jodwasserstoffsäure, oder Sulfonsäureester, z.B. p-Toluolsulfonsäureester. Als Kondensationsmittel können beispielsweise Alkalialkanolate, z.B. Natriummethylat oder Natriumäthylat, oder Alkalimetallhydride, z.B. Natriumhydrid, oder Metallamide, wie Natriumamid oder Lithiumamid, verwendet werden. Als Lösungsmittel eignen sich indifferente Lösungsmittel, z.B. Kohlenwasserstoffe, wie Benzol oder Toluol, oder, falls Alkanolate als Kondensationsmittel verwendet werden, entsprechende Alkanole. Die Reaktionstemperaturen liegen vorzugsweise zwischen 20 und 130°C.

Ferner kann gegebenenfalls eine Verbindung der Formel I, in welcher X einen zweiwertigen Rest der Teilformel

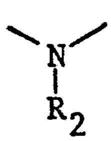

(Ia)

bedeutet, und worin $R_2$ Wasserstoff darstellt, in ein

anderes Verfahrensprodukt übergeführt werden, dessen Rest $R_2$ Niederalkyl darstellt.

Diese Umwandlung wird vorzugsweise so vorgenommen, dass man das obengenannte Verfahrensprodukt in Gegenwart von Lösungs- und basischen Kondensationsmitteln mit einem reaktionsfähigen Niederalkylester umsetzt.

Als reaktionsfähige Ester können z.B. Niederalkylhalogenide, wie -chloride oder -bromide, ferner Sulfonsäureester, wie der p-Toluolsulfonsäure-methylester oder -äthylester, oder Schwefelsäureester, wie z.B. das Dimethyl- oder Diäthylsulfat, verwendet werden. Als basische Kondensationsmittel eignen sich Alkalimetallalkanolate, wie Kalium-tert.-butylat, oder entsprechende Amide, wie Natriumamid, oder Metallhydride, wie Natrium- oder Lithiumhydrid. Geeignete Lösungsmittel sind solche Lösungsmittel, die unter den Reaktionsbedingungen inert sind, beispielsweise Kohlenwasserstoffe, wie Benzol oder Toluol, ferner ätherartige Lösungsmittel, z.B. Tetrahydrofuran, Dioxan, Aethylenglykoldimethyläther, oder Amide, wie Phosphorsäure-hexamethyltriamid oder Dimethylformamid.

Je nach den Verfahrensbedingungen und Ausgangsstoffen erhält man die Endstoffe gegebenenfalls in freier Form oder in Form ihrer Salze, die sich in üblicher Weise ineinander oder in andere Salze umwandeln lassen. So kann man freie Verbindungen der Formel I aus erhaltenen Säureadditionssalzen, z.B. durch Behandeln mit Basen oder basischen Ionenaustauschern bilden, während man freie Basen der Formel I z.B. durch Umsetzen mit organischen oder anorganischen Säuren, insbesondere solchen, die zur Bildung pharmazeutisch verwendbarer Salze geeignet sind, wie den obgenannten, in Säureadditionssalze überführen kann.

Salze der neuen Verbindungen können auch zu Reinigungszwecken verwendet werden, z.B. indem man die freien Verbindungen in ihre Salze überführt, diese isoliert und gegebenenfalls reinigt, und wieder in die freien Verbindungen überführt. Infolge der engen Beziehungen zwischen den neuen Verbindungen in freier Form und in Form ihrer Salze sind im Vorausgegangenen und nachfolgend unter den freien Verbindungen sinn- und zweckmässig gegebenenfalls auch die entsprechenden Salze zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Verfahrensschritte durchführt, oder das Verfahren auf irgeneiner Stufe abbricht, oder bei denen man einen Ausgangsstoff unter den Reaktionsbedingungen bildet, oder bei denen eine Reaktionskomponente gegebenenfalls in Form ihrer Salze vorliegt.

Zweckmässig verwendet man für die Durchführung des erfindungsgemässen Verfahrens solche Ausgangsstoffe, die zu den eingangs besonders erwähnten Gruppen von Endstoffen und besonders zu den speziell beschriebenen oder hervorgehobenen Endstoffen führen.

Die neuen Verbindungen können z.B. in Form pharmazeutischer Präparate Verwendung finden, welche eine wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln, welche den Wirkstoff zu-

sammen mit Verdünnungsmitteln, z.B. Laktose, Dextrose, Sukrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel. Ferner kann man die neuen pharmakologisch wirksamen Verbindungen in Form von injizierbaren, z.B. intravenös, verabreichbaren Präparaten oder von Infusionslösungen verwenden. Solche Lösungen sind vorzugsweise isotonische wässerige Lösungen oder Suspensionen, wobei diese z.B. als lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1% bis 100%; insbesondere von etwa 1% bis etwa 50%, Lyophilisate bis zu 100% des Aktivstoffes. Die Dosierung hängt von der Applikationsweise, der Spezies, dem Alter und von dem individuellen Zustand ab.

Die täglichen Dosen der freien Basen oder von pharmazeutisch annehmbaren Salzen derselben bewegen sich zwischen etwa 0,01 mg/kg und 1 mg/kg für Warmblüter im allgemeinen und etwa 0,001 g bis etwa 0,025 g für Warmblüter mit einem Gewicht von etwa 70 kg.

Die folgenden Beispiele dienen zur Illustration der Erfindung; Temperaturen werden in Celsiusgraden angegeben.

Beispiel 1

42,1 g (0,1 Mol) 2-Cyano-10,11-bis-brommethyl-dibenzo[b,f]thiepin werden in 250 ml absolutem Benzol gelöst und innerhalb einer Stunde bei 40° zu einer Lösung von 62 g (2 Mol) Methylamin in 500 ml Methanol getropft. Man rührt das Reaktionsgemisch noch zwei Stunden bei 50° und destilliert anschliessend das Lösungsmittel und das überschüssige Methylamin ab. Den Rückstand versetzt man mit Wasser und extrahiert die erhaltene Suspension mit Aether. Die organische Phase wird abgetrennt, mit Wasser gewaschen, über Kaliumcarbonat getrocknet und eingeengt. Beim Abkühlen kristallisiert das 2-Methyl-5-cyano-2,3-dihydro-1H-dibenzo[2,3:6,7]thiepino[4,5-c]pyrrol vom Smp. 132-136° aus.

14,5 g (0,05 Mol) der erhaltenen Base werden in 200 ml Aceton gelöst und danach werden vorsichtig 4,8 g (0,05 Mol) Methansulfonsäure zugefügt, worauf das Methansulfonat auskristallisiert, welches nach dem Umkristallisieren aus absolutem Aethanol bei 263-266° schmilzt.

Das Ausgangsmaterial kann auf folgende Weise hergestellt werden:

Zu einer Lösung von 30,5 g 8-Brom-10,11-dihydro-dibenz[b,f]thiepin-10-on in 200 ml absolutem Benzol wird 4,3 g Natriumamid (als 2%-ige Suspension in Toluol) innerhalb 30 Minuten zugegeben. Dann wird die Mischung zwei Stunden unter Rückfluss gekocht, auf 50° abgekühlt und tropfenweise mit 19,9 g Methyljodid versetzt. Anschliessend wird die Suspension 20 Stunden bei 50° gerührt, abgekühlt und mit 200 ml Wasser versetzt. Die

organische Phase wird abgetrennt, mit 50 ml Wasser gewaschen, über Natriumsulfat getrocknet und unter vermindertem Druck zur Trockne eingedampft. Den Rückstand kristallisiert man bei Raumtemperatur aus 70 ml absolutem Aether. Das 8-Brom-11-methyl-10,11-dihydro-dibenz [b,f]thiepin-10-on schmilzt bei 124-126°.

Zu einer Grignardlösung, die durch Zugabe von 10,2 g Methyljodid zu 1,76 g Magnesium in 17,5 ml absolutem Aether hergestellt wurde, tropft man unter Durchleiten von Stickstoff bei 0° eine Lösung von 11,5 g 8-Brom-11-methyl-10,11-dihydro-dibenz[b,f]thiepin-10-on in 50 ml absolutem Benzol zu. Anschliessend wird die Mischung 16 Stunden gerührt und auf 200 ml Eiswasser gegossen. Das ausgeschiedene Oel wird in 100 ml Aether gelöst, die Aetherlösung mit 20 ml Wasser gewaschen, über Magnesiumsulfat getrocknet und unter vermindertem Druck eingeengt. Nach Zusatz von wenig Petroläther wird das 2-Brom-10,11-dimethyl-11-hydro-10,11-dihydro-dibenz[b,f] thiepin als farblose Kristalle erhalten, F. 96-98°.

Eine Suspension von 7,7 g 2-Brom-10,11-dimethyl-11-hydroxy-10,11-dihydro-dibenz[b,f]thiepin in 40 ml 20%-iger Schwefelsäure wird unter schnellem Rühren 36 Stunden unter Rückfluss gekocht. Dann kühlt man die Mischung ab und extrahiert sie dreimal mit je 50 ml Aether. Die Aetherextrakte werden vereinigt, mit Wasser neutral gewaschen, über Magnesiumsulfat getrocknet und unter vermindertem Druck zur Trockne eingedampft. Man löst den Rückstand in 32 ml 20%-iger äthanolischer Kaliumhydroxydlösung und erhitzt die Lösung 40 Stunden unter Rückfluss. Hierauf engt man sie unter vermindertem Druck zur Trockne ein und extrahiert den Rückstand mit 100 ml Aether. Die Aetherlösung wird mit 20 ml Wasser

gewaschen, über Magnesiumsulfat getrocknet und unter vermindertem Druck zur Trockne eingedampft. Man löst den Rückstand in 300 ml Methanol, filtriert von wenig Unlöslichem ab und engt das Filtrat unter vermindertem Druck ein. Das 2-Brom-10,11-dimethyl-dibenz[b,f]thiepin wird dabei als gelbliche Kristalle erhalten, F. 108-111°.

31,7 g (0,1 Mol) 2-Brom-10,11-dimethyl-dibenzo [b,f]thiepin, 10,7 g (0,12 Mol) Kupfer-I-cyanid und 15 ml Dimethylformamid werden unter Rühren bei einer Badtemperatur von 200° 3 Stunden erhitzt. Anschliessend wird das Reaktionsgemisch auf 40° abgekühlt und mit 200 ml einer 50%-igen wässerigen Aethylendiaminlösung und 200 ml Methylenchlorid 2 Stunden stark gerührt. Dann wird die organische Phase abgetrennt und die wässerige Phase noch zwei mal mit 100 ml Methylenchlorid extrahiert. Die vereinigten organischen Lösungen werden mit Wasser gewaschen, über Natriumsulfat getrocknet und eingedampft. Der Rückstand, das 2-Cyano-10,11-dimethyl-dibenz[b,f]thiepin wird aus Methanol umkristallisiert und schmilzt bei 134-135°.

26,3 g (0,1 Mol) 2-Cyano-10,11-dimethyl-dibenzo[b,f]thiepin werden in 525 ml Tetrachlorkohlenstoff gelöst, und diese Lösung wird mit 35,6 g (0,2 Mol) N-Bromsuccinimid versetzt. Unter Rühren in einer Stickstoffatmosphäre wird unter Belichten mit einer UV-Lampe das Gemisch zum Sieden erhitzt. Man hält das Gemisch solange am Sieden, bis alles N-Bromsuccinimid, welches auf dem Boden des Gefässes liegt, sich in auf der Lösung schwimmendes Succinimid umgewandelt hat; Dauer etwa 10 Minuten. Hierauf kühlt man das Reaktionsgemisch auf 20° ab und trennt das Succinimid durch Filtration ab. Das Filtrat wird mit Wasser gewaschen, über Natrium-

sulfat getrocknet und im Vakuum eingeengt. Beim Abkühlen kristallisiert das 2-Cyano-10,11-bis-brommethyl-dibenzo [b,f]thiepin vom Smp. 165-167° aus.

## Beispiel 2

Analog Beispiel 1 können unter Verwendung der entsprechenden Ausgangsstoffe hergestellt werden:

a) aus 42,1 g (0,1 Mol) 2-Cyano-10,11-bis-brommethyl-dibenzo[b,f]thiepin und 90 g (2 Mol) Aethylamin in 500 ml Methanol das 2-Aethyl-5-cyano-2,3-dihydro-1H-dibenzo[2,3:6,7]thiepino[4,5-c]pyrrol, Smp. 129-131° (aus Acetonitril); Methansulfonat Smp. 258-260° (aus abs. Aethanol);

b) aus 42,1 g (0,1 Mol) 2-Cyano-10,11-bis-brommethyl-dibenzo[b,f]thiepin und 49,5 g (0,5 Mol) (Aminomethyl)-cyclopentan in 300 ml Methanol das 2-(Cyclopentylmethyl)-5-cyano-2,3-dihydro-1H-dibenzo[2,3:6,7] thiepino[4,5-c]pyrrol, Smp. 97-100° (aus Acetonitril); Methansulfonat Smp. 162-168° (aus abs. Aethanol);

c) aus 42,1 g (0,1 Mol) 2-Cyano-10,11-bis-brommethyl-dibenzo[b,f]thiepin und 59 g (1 Mol) Propylamin in 250 ml Methanol das 2-Propyl-5-cyano-2,3-dihydro-1H-dibenzo[2,3:6,7]thiepino[4,5-c]pyrrol, Smp. 130-131° (aus Diäthyläther); Methansulfonat Smp. 135-137° (aus abs. Aethanol/abs. Diäthyläther);

d) aus 42,1 g (0,1 Mol) 2-Cyano-10,11-bis-brommethyl-dibenzo[b,f]thiepin und 37,5 g (0,5 Mol) 3-Aminopropanol in 250 ml Methanol das 5-Cyano-1,3-dihydro-2H-dibenzo[2,3:6,7]thiepino[4,5-c]pyrrol-2-propanol, Rohprodukt; Hydrochlorid Smp. 273-276° (aus Methanol).

Beispiel 3

Analog Beispiel 1 kann unter Verwendung der entsprechenden Ausgangsstoffe hergestellt werden:

Aus 40,5 g (0,1 Mol) 2-Cyano-10,11-bis-brommethyl-dibenz[b,f]oxepin und 90 g (2 Mol) Aethylamin in 500 ml Methanol das 2-Aethyl-5-cyano-2,3-dihydro-1H-dibenz[2,3:6,7]oxepino[4,5-c]pyrrol, Smp. 125-127° (aus Diäthyläther); Methansulfonat Smp. 199-201° (aus abs. Aethanol/Aceton).

Das Ausgangsmaterial kann auf folgende Weise hergestellt werden:

Ein Gemisch von 560,0 g 2-(4-Brom-phenyloxy)-benzoesäure in 1940 ml absolutem Benzol, 218 ml absolutem Aethanol und 32,6 ml konzentrierter Schwefelsäure wird 32 Stunden unter Rückfluss gekocht, wobei man das sich bildende Wasser in einem Wasserabscheider entfernt. Man kühlt das Reaktionsgemisch auf 10° ab und wäscht unter Zusatz von Eis mit 1000 ml Wasser, 500 ml einer 2-n. wässerigen Natriumcarbonatlösung und anschliessend nochmals mit 1000 ml Wasser. Die organische Phase wird abgetrennt, über Magnesiumsulfat getrocknet und unter einem Druck von 11 mm Hg bei 40° eingedampft. Der Rückstand wird im Hochvakuum destilliert und ergibt den 2-(4-Brom-phenyloxy)-benzoesäure-äthylester, Kp. 140-150°/0,05 mm Hg.

Eine Lösung von 477,0 g des 2-(4-Brom-phenyloxy)-benzoesäure-äthylesters in 900 ml absolutem Diäthyläther wird innerhalb 1 Stunde und unter Durchleiten eines Stickstoffstroms zu 42,3 g Lithiumaluminiumhydrid in 500 ml Diäthyläther eingetropft. Das Reaktionsgemisch wird 6 Stunden unter Rückfluss gekocht, auf 0-5° abgekühlt und unter Durchleiten von Stickstoff mit 450 ml Essigsäureäthylester und anschliessend vorsichtig mit

350 ml Wasser versetzt. Man filtriert vom Niederschlag ab und wäscht diesen mit Diäthyläther nach. Die wässerige Phase des Filtrats wird abgetrennt und mit 100 ml Diäthyläther gewaschen; die vereinigten organischen Phasen werden über Magnesiumsulfat getrocknet und unter 11 mm Hg zur Trockne eingedampft; der 2-(4-Brom-phenyloxy)-benzylalkohol bleibt als farbloses Oel zurück.

Ein Gemisch von 413,0 g des 2-(4-Brom-phenyloxy)-benzylalkohols und 1290 ml 48%-iger Bromwasserstoffsäure wird 4 Stunden unter Rückfluss gekocht, dann abgekühlt und auf 2000 ml Eis und Wasser ausgegossen. Das ausgeschiedene, grünliche Oel wird in 2000 ml Diäthyläther gelöst. Die organische Phase wird zweimal mit 400 ml Wasser und mit 400 ml einer 1-n. wässerigen Natriumhydrogencarbonatlösung gewaschen, über Magnesiumsulfat getrocknet und unter 11 mm Hg bei 40° eingedampft. Das als öliger Rückstand erhaltene 2-(4-Bromphenyloxy)-benzylbromid wird ohne Reinigung weiterverwendet.

Man fügt 457,8 g des rohen 2-(4-Brom-phenyloxy)-benzylbromids innerhalb einer Stunde zu einem unter Rückfluss siedenden Gemisch von 171,0 g Natriumcyanid in 160 ml Wasser und 44 ml Aethanol; gleichzeitig werden 362 ml Aethanol zugetropft. Anschliessend kocht man 3 weitere Stunden unter Rückfluss und verdünnt dann mit 1500 ml Wasser. Man wäscht die wässerig-äthanolische Phase mit 1000 ml Diäthyläther, trennt die Aetherphase ab, wäscht sie zweimal mit 200 ml Wasser, trocknet sie über Magnesiumsulfat und engt sie unter 11 mm Hg zur Trockne ein. Den Rückstand kristallisiert man aus einem Gemisch von Diäthyläther und Petroläther und erhält so das 2-(4-Bromphenyloxy)-phenylacetonitril, F. 56-58°.

25,3 g Natrium werden unter Rühren in 400 ml abs. Aethanol gelöst, und anschliessend destilliert man wieder ca. 200 ml abs. Aethanol aus dem Reaktionsgemisch ab. Hierauf gibt man 1500 ml abs. Toluol zu und destilliert unter einer Vigreuxkolonne weiter, bis der Siedepunkt 108° erreicht hat, wobei das Natriumäthylat auskristallisiert. Bei 100-110° wird nun innerhalb einer Stunde ein Gemisch von 288 g (1 Mol) 2-(4-Bromphenyloxy)-phenylacetonitril und 354 g (3 Mol) Diäthylcarbonat unter gleichzeitigem Abdestillieren des entstehenden Aethanols zugetropft. Nach Beendigung des Zutropfens wird das Reaktionsgemisch weiter destilliert bis der Siedepunkt wieder 108-110° erreicht hat. Anschliessend wird das Reaktionsgemisch auf Raumtemperatur abgekühlt, mit 300 ml abs. Toluol verdünnt und innerhalb einer Stunde 170 g (1,2 Mol) Methyljodid zugetropft. Zur vollständigen Umsetzung wird das Gemisch 1 Stunde bei Raumtemperatur und 5 Stunden bei 80° weitergerührt. Nach dem Abkühlen auf Raumtemperatur lässt man 1 Liter Wasser zulaufen, trennt die organische Phase ab, wäscht sie mit Wasser und dampft sie nach dem Trocknen über Natriumsulfat im Vakuum vollständig ein. Als Rückstand bleibt der rohe 2-(4-Bromphenyloxy)-phenyl-α-methyl-cyanessigsäure-äthylester zurück.

Unter gutem Rühren werden 374 g (1 Mol) roher 2-(4-Bromphenyloxy)-phenyl-α-methyl-cyanessigsäure-äthylester, 830 ml Aethanol 96%-ig und 460 ml 50%-ige wässerige Kalilauge 24 Stunden unter Rückfluss gekocht. Dann wird das Reaktionsgemisch unter 11 mm Hg bei 50° eingedampft und der Rückstand in 3500 ml Wasser gelöst. Nach dem Klarfiltrieren wird die alkalische Lösung mit konzentrierter Salzsäure angesäuert, wobei die 2-(4-Bromphenyloxy)-phenyl-α-methylessigsäure auskristalli-

siert. Nach dem Abfiltrieren wird die erhaltene Säure im Vakuum bei 50° getrocknet und anschliessend aus Acetonitril umkristallisiert, Smp. 99 - 101°

321 g (1 Mol) 2-(4-Bromphenyloxy)-phenyl-α-methylessigsäure und 3210 g Polyphosphorsäure werden unter gutem Rühren eine Stunde auf 100 - 105° erwärmt. Anschliessend giesst man das Reaktionsgemisch unter Rühren in 3 Liter Wasser, wobei durch Eiszugabe die Temperatur unter 10° gehalten wird. Das ausgeschiedene Oel wird mit Diäthyläther extrahiert, die organische Phase wird mit Wasser gewaschen, über Kaliumcarbonat getrocknet und eingeengt, wobei nach dem Abkühlen das 8-Brom-11-methyl-dibenz[b,f]oxepin-10(11H)-on vom Smp. 85-87° auskristallisiert.

Zu einer Grignardlösung, die aus 49 g (2 Mol) Magnesium, 450 ml abs. Aether und 284 g Methyljodid bereitet wird, lässt man innerhalb 5 Stunden unter gutem Rühren eine Lösung von 303 g (1 Mol) 8-Brom-11-methyl-dibenz[b,f]oxepin-10(11H)-on in 1500 ml abs. Toluol eintropfen, wobei man eine Reaktionstemperatur von -5 bis 0° einhält. Anschliessend erwärmt man das Reaktionsgemisch auf 55° und rührt es 15 Stunden bei dieser Temperatur weiter. Hierauf kühlt man das Reaktionsgemisch auf 0° ab und giesst es unter Rühren auf eine Lösung von 680 g Ammoniumchlorid in 2000 ml Eiswasser. Die organische Phase wird abgetrennt und die wässerige Phase mit Toluol extrahiert. Die vereinigten organischen Lösungen wäscht man mit Wasser, trocknet sie über Natriumsulfat und dampft sie im Vakuum ein. Als Rückstand bleibt das 8-Brom-10,11-dimethyl-dihydrodibenz[b,f]oxepin-10-ol als Oel zurück.

319 g (1 Mol) 8-Brom-10,11-dimethyl-dihydro-dibenz[b,f]oxepin-10-ol (Rohprodukt) und 1,5 g p-Toluolsulfosäure werden in einer Destillationsapparatur unter 11 mm Hg 1 Stunde auf 180° und 5 Stunden auf 200° Aussentemperatur erhitzt, wobei sich Wasser abspaltet. Anschliessend wird die Destillationsvorlage ausgewechselt und das entstandene 2-Brom-10,11-dimethyl-dibenz[b,f]oxepin im Hochvakuum destilliert, Kp 142-148°/0,01 Torr. Das hellgelbe Destillat löst man in 300 ml Acetonitril und kühlt die Lösung auf 0° ab, wobei das Produkt auskristallisiert, Smp. 117-119°.

30,1 g (0,1 Mol) 2-Brom-10,11-dimethyl-dibenz[b,f]oxepin, 10,7 g (0,12 Mol) Kupfer-I-cyanid und 15 ml Dimethylformamid werden unter Rühren bei einer Badtemperatur von 200° 3 Stunden erhitzt. Anschliessend wird das Reaktionsgemisch auf 40° abgekühlt und mit 200 ml einer 50%-igen wässerigen Aethylendiaminlösung und 200 ml Methylenchlorid 2 Stunden stark gerührt. Dann wird die organische Phase abgetrennt und die wässerige Phase noch zwei mal mit 100 ml Methylenchlorid extrahiert. Die vereinigten organischen Lösungen werden mit Wasser gewaschen, über Natriumsulfat getrocknet und eingedampft. Der Rückstand, das 2-Cyano-10,11-dimethyl-dibenz[b,f]oxepin wird aus Hexan umkristallisiert und schmilzt bei 112-114°.

24,7 g (0,1 Mol) 2-Cyano-10,11-dimethyl-dibenz[b,f]oxepin werden in 525 ml Tetrachlorkohlenstoff gelöst und diese Lösung mit 35,6 g (0,2 Mol) N-Bromsuccinimid versetzt. Das Gemisch wird unter Rühren in einer Stickstoffatmosphäre unter Belichten mit einer UV-Lampe zum Sieden erhitzt. Man hält das Gemisch solange am Sieden, bis alles N-Bromsuccinimid, welches auf dem Boden des Gefasses liegt, sich in auf der Lösung schwimmendes Succinimid umgewandelt hat; Dauer etwa 5 Minuten. Hierauf kühlt

man das Reaktionsgemisch auf 20° ab und trennt das Succinimid durch Filtration ab. Das Filtrat wird mit Wasser gewaschen, über Natriumsulfat getrocknet und im Vakuum eingeengt. Beim Abkühlen kristallisiert das 2-Cyano-10,11-bis-brommethyl-dibenz[b,f]oxepin vom Smp. 176-178° aus.

Beispiel 4

Zu einer Lösung von 16,7 g (0,05 Mol) 5-Cyano-1,3-dihydro-2H-dibenzo[2,3:6,7]thiepino[4,5-c]pyrrol-2-propanol (hergestellt nach Beispiel 2d), in 50 ml absolutem Pyridin werden innerhalb 15 Minuten unter Rühren 12,2 g (0,075 Mol) Octanoylchlorid zugetropft, wobei die Temperatur zwischen 0° und 5° gehalten wird. Anschliessend rührt man 20 Stunden bei Raumtemperatur weiter. Hierauf giesst man das Reaktionsgemisch auf Eiswasser und extrahiert es mit Aether. Die organische Phase wird abgetrennt, mit Wasser gewaschen und nach dem Trocknen über Natriumsulfat eingedampft. Der ölige Rückstand, das 2-(3-Octanoyloxypropyl)-5-cyano-2,3-dihydro-1H-dibenzo[2,3:6,7]thiepino[4,5-c]pyrrol, wird im Hochvakuum bei 90° 0,01 Torr. bis zur Gewichtskonstanz erwärmt.

21,4 g Rohbase werden in 150 ml Aceton gelöst und mit einer Lösung von 4,2 g (0,047 Mol) wasserfreier Oxalsäure in 22 ml absolutem Aethanol versetzt, worauf das 2-(3-Octanoyloxypropyl)-5-cyano-2,3-dihydro-1H-dibenzo[2,3:6,7]thiepino[4,5-c]pyrrol-oxalat auskristallisiert, das nach dem Umkristallisieren aus absolutem Aethanol bei 188-189° schmilzt.

Beispiel 5

Analog Beispiel 4 können unter Verwendung der entsprechenden Ausgangsstoffe hergestellt werden:

Aus 16,7 g (0,05 Mol) 5-Cyano-1,3-dihydro-2H-dibenzo[2,3:6,7]thiepino[4,5-c]pyrrol-2-propanol in 50 ml absolutem Pyridin und 5,9 g (0,075 Mol) Acetylchlorid das 2-(3-Acetoxypropyl)-5-cyano-2,3-dihydro-1H-dibenzo[2,3:6,7]thiepino[4,5-c]pyrrol, Smp. 101-105° (aus Acetonitril); Hydrochlorid Smp. 180-183° zers. (aus abs. Aethanol).

Beispiel 6

Ein Gemisch von 19,7 g (0,05 Mol) 5-Brom-2-(cyclopentylmethyl)-2,3-dihydro-1H-dibenzo[2,3:6,7]thiepino[4,5-c]pyrrol, 10,7 g (0,12 Mol) Kupfer-I-cyanid in 20 ml Dimethylformamid wird in einer Stickstoffatmosphäre 22 Stunden unter Rühren auf 180° erwärmt. Hierauf wird auf 30° abgekühlt, mit 100 ml Methylenchlorid verdünnt und mit 50 ml einer 50%-igen wässerigen Aethylendiaminlösung versetzt. Die organische Phase wird anschliessend abgetrennt, mit Wasser gewaschen und nach dem Trocknen über Natriumsulfat eingedampft. Der kristalline Rückstand, das 5-Cyano-2-(cyclopentylmethyl)-2,3-dihydro-1H-dibenzo[2,3:6,7]thiepino[4,5-c]pyrrol schmilzt nach dem Umkristallisieren aus Acetonitril bei 97-100°.

Zur Ueberführung ins Methansulfonat löst man 10 g (0,03 Mol) Base in 50 ml Aceton und versetzt diese Lösung unter Rühren mit 2,88 g (0,03 Mol) Methansulfonsäure, worauf das 5-Cyano-2-(cyclopentylmethyl)-2,3-dihydro-1H-dibenzo[2,3:6,7]thiepino[4,5-c]pyrrol-methansulfonat vom Smp. 162-168° auskristallisiert.

Das Ausgangsmaterial kann auf folgende Weise hergestellt werden: 47,5 g (0,1 Mol) 2-Bromo-10,11-bis-brommethyldibenzo[b,f]thiepin werden in 250 ml absolutem Benzol gelöst und innerhalb einer Stunde bei 40° zu einer Lösung von 49,5 g (0,5 Mol) (Aminomethyl)-cyclopentan in 500 ml Methanol getropft. Man rührt das Reaktionsgemisch noch zwei Stunden bei 50° und destilliert anschliessend das Lösungsmittel ab. Den Rückstand versetzt man mit Wasser und extrahiert die erhaltene Suspension mit Aether. Die organische Phase wird abgetrennt, mit Wasser gewaschen, über Kaliumcarbonat getrocknet und eingeengt. Beim Abkühlen kristallisiert das 2-(Cyclopentylmethyl)-5-bromo-2,3-dihydro-1H-dibenzo[2,3:6,7]thiepino[4,5-c]pyrrol aus.

Beispiel 7

Tabletten, enthaltend 0,002 g des Methansulfonsäuresalzes des 2-Methyl-5-cyano-2,3-dihydro-1H-dibenzo [2,3:6,7]thiepino[4,5-c]pyrrol, werden wie folgt hergestellt:

Zusammensetzung (für 10000 Tabletten):

| | |
|---|---|
| Methansulfonsäuresalz des 2-Methyl-5-cyano-2,3-dihydro-1H-dibenzo[2,3:6,7]thiepino[4,5-c]pyrrol | 20,00 g |
| Lactose | 250,80 g |
| Kartoffelstärke | 434,70 g |
| Stearinsäure | 10,00 g |
| Talk | 250,00 g |
| Magnesiumstearat | 2,50 g |
| kolloidales Siliciumdioxid | 32,00 g |
| Aethanol | q.s. |

Ein Gemisch des Methansulfonsäuresalzes des 2-Methyl-5-cyano-2,3-dihydro-1H-dibenzo[2,3:6,7]thiepino[4,5-c]pyrrol, der Lactose und 194,70 g Kartoffelstärke wird mit einer äthanolischen Lösung der Stearinsäure befeuchtet und durch ein Sieb granuliert. Nach dem Trocknen mischt man die restliche Kartoffelstärke, den Talk, das Magnesiumstearat und das kolloidale Silicimdioxid zu und presst die Mischung zu Tabletten von je 0,1 g Gewicht, die gewünschtenfalls mit Teilkerben zur feineren Anpassung der Dosierung versehen sein können.

Beispiel 8

Dragées, enthaltend 0,0005 g des Methansulfonsäuresalzes des 2-Aethyl-5-cyano-2,3-dihydro-1H-dibenzo[2,3:6,7]thiepino[4,5-c]pyrrol, werden wie folgt hergestellt:

Zusammensetzung (für 10 000 Dragées):

| | |
|---|---|
| Methansulfonsäuresalz des 2-Aethyl-5-cyano-2,3-dihydro-1H-dibenzo[2,3:6,7]thiepino[4,5-c]pyrrol | 5,00 g |
| Lactose | 180,00 g |
| Stearinsäure | 10,00 g |
| kolloidales Siliciumdioxid | 60,00 g |
| Talk | 180,00 g |
| Kartoffelstärke | 20,00 g |
| Magnesiumstearat | 2,50 g |
| Saccharose (krist.) | 524,78 g |
| Schellack | 6,00 g |
| arabische Gummi | 10,00 g |
| Farbstoff | 0,22 g |
| Titandioxid | 1,50 g |
| Aethanol | q.s. |

Aus dem 2-Aethyl-5-cyano-2,3-dihydro-1H-dibenzo[2,3:6,7]thiepino[4,5-c]pyrrol-methansulfonat, der Lactose und einer äthanolischen Lösung der Stearinsäure stellt man ein Granulat her, das man nach dem Trocknen mit dem kolloidalen Siliciumdioxid, dem Talk, der Kartoffelstärke und dem Magnesiumstearat vermischt und zu Dragée-Kernen presst. Diese werden anschliessend mit einem konzentrierten Sirup aus der Saccharose, dem Schellack, dem arabischen Gummi, dem Farbstoff und dem Titandioxid überzogen und getrocknet. Man erhält so Dragées mit einem Gewicht von je 0,100 g.

Beispiel 9

Kapseln, enthaltend 0,002 g des Methansulfonsäuresalzes des 2-(Cyclopentylmethyl)-5-cyano-2,3-dihydro-1H-dibenzo[2,3:6,7]thiepino[4,5-c]pyrrol, werden wie folgt hergestellt:

Zusammensetzung (für 1 000 Kapseln)

| | |
|---|---|
| Methansulfonsäuresalz des 2-(Cyclopentylmethyl)-5-cyano-2,3-dihydro-1H-dibenzo[2,3:6,7]thiepino[4,5-c]pyrrol | 2,0 g |
| Lactose | 253,0 g |
| Gelatine | 2,0 g |
| Maisstärke | 10,0 g |
| Talk | 15,0 g |
| Wasser | q.s. |

Man mischt das Methansulfonsäuresalz des 2-(Cyclopentylmethyl)-5-cyano-2,3-dihydro-1H-dibenzo[2,3:6,7]thiepino[4,5-c]pyrrol mit der Lactose, befeuchtet die Mischung gleichmässig mit einer wässerigen Lösung der Gelatine und granuliert sie durch ein geeignetes Sieb (z.B. ein Sieb mit lichter Maschenweite von 1,2-1,5 mm).Das Granulat mischt man mit der getrockneten Maisstärke und dem Talk und füllt es gleichmässig in die Hartgelatinekapseln (Grösse 1) ab.

Beispiel 10

Eine wässerige Injektionslösung, enthaltend 0,001 g/ml des Methansulfonsäuresalzes des 2-(Cyclopentylmethyl)-5-cyano-2,3-dihydro-1H-dibenzo[2,3:6,7]thiepino[4,5-c]pyrrol wird wie folgt hergestellt:

Zusammensetzung (für 1000 Ampullen)

| | |
|---|---|
| Methansulfonsäuresalz des 2-(Cyclopentylmethyl)-5-cyano-2,3-dihydro-1H-dibenzo[2,3:6,7]thiepino[4,5-c]pyrrol | 1,00 g |
| Wasser | q.s. |

Eine Lösung des Methansulfonsäuresalzes des 2-(Cyclopentylmethyl)-5-cyano-2,3-dihydro-1H-dibenzo[2,3:6,7]thiepino[4,5-c]pyrrol in 1000 ml Wasser wird in Ampullen abgefüllt und sterilisiert. Eine Ampulle enthält eine 0,1%-ige Lösung des Wirkstoffs.

Patentansprüche

1. Azatetracyclische Carbonitrile der Formel I

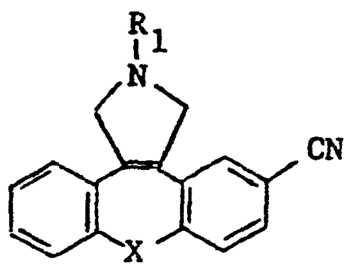

(I)

in welcher $R_1$ Wasserstoff, Niederalkyl, Cycloalkylniederalkyl, Niederalkenyl, freies, veräthertes oder verestertes Hydroxyniederalkyl ist, und X Epoxy, Epithio, Methylen, oder einen zweiwertigen Rest der Teilformel

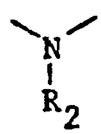

(Ia)

bedeutet, in der $R_2$ Wasserstoff oder Niederalkyl darstellt, sowie Salze von solchen Verbindungen.

2. Verbindungen der im Anspruch 1 angegebenen Formel I, worin $R_1$ Wasserstoff, Niederalkyl, Cycloalkylniederalkyl mit 4-8 Kohlenstoffatomen, Niederalkenyl, Hydroxyniederalkyl, Niederalkoxyniederalkyl, oder Alkanoyloxy-niederalkyl bedeutet und X Epoxy, Epithio, Methylen oder den zweiwertigen Rest der Teilformel Ia bedeutet,

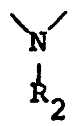

(Ia)

in welcher $R_2$ für Wasserstoff oder Niederalkyl mit bis 4 Kohlenstoffatomen steht, und deren Salze.

3. Verbindungen der im Anspruch 1 angegebenen Formel I, worin $R_1$ Wasserstoff, Niederalkyl, Cycloalkylniederalkyl mit 4-8 Kohlenstoffatomen, Niederalkenyl oder Hydroxyniederalkyl, X Epoxy oder Epithio darstellt, und deren pharmazeutisch annehmbare Salze.

4. Verbindungen der im Anspruch 1 angegebenen Formel I, worin $R_1$ Wasserstoff, Methyl, Aethyl, Propyl, Cyclopentylmethyl oder 3-Hydroxypropyl, und X Epoxy oder Epithio bedeutet, und deren pharmazeutisch annehmbare Salze.

5. 2-Methyl-5-cyano-2,3-dihydro-1H-dibenzo[2,3:6,7]-thiepino[4,5-c]pyrrol und deren pharmazeutisch annehmbare Salze.

6. 2-(Cyclopentylmethyl)-5-cyano-2,3-dihydro-1H-dibenzo[2,3:6,7]thiepino[4,5-c]pyrrol und deren pharmazeutisch annehmbare Salze.

7. 5-Cyano-1,3-dihydro-2H-dibenzo[2,3:6,7]thiepino-[4,5-c]pyrrol-2-propanol und deren pharmazeutisch annehmbare Salze.

8. 2-Aethyl-5-cyano-2,3-dihydro-1H-dibenzo[2,3:6,7]-thiepino[4,5-c]pyrrol und deren pharmazeutisch annehmbare Salze.

9. 2-Propyl-5-cyano-2,3-dihydro-1H-dibenzo[2,3:6,7]-thiepino[4,5-c]pyrrol und deren pharmazeutisch annehmbare Salze.

10. 2-Aethyl-5-cyano-2,3-dihydro-1H-dibenz[2,3:6,7]oxepino[4,5-c]pyrrol und deren pharmazeutisch annehmbare Salze.

11. 2-(3-Octanoyloxypropyl)-5-cyano-2,3-dihydro-1H-dibenzo[2,3:6,7]thiepino[4,5-c]pyrrol und deren pharmazeutisch annehmbare Salze.

12. 2-(3-Acetoxypropyl)-5-cyano-2,3-dihydro-1H-dibenzo[2,3:6,7]thiepino[4,5-c]pyrrol und deren pharmazeutisch annehmbare Salze.

13. Die Verbindungen der Ansprüche 1-12 zur Verwendung als Pharmazeutika.

14. Pharmazeutische Präparate enthaltend eine der in den Ansprüchen 1-12 beanspruchten Verbindungen und mindestens einen pharmazeutischen Trägerstoff.

15. Verwendung der Verbindungen der Ansprüche 1-12 zur Herstellung von pharmazeutischen Präparaten.

16. Verfahren zur Herstellung von azatetracyclischen Carbonitrilen der Formel I

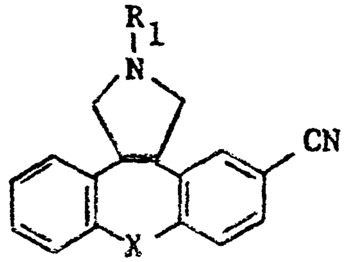

(I)

in welcher $R_1$ Wasserstoff, Niederalkyl, Cycloalkylniederalkyl, Niederalkenyl, freies, veräthertes oder verestertes Hydroxyniederalkyl ist, und X Epoxy, Epithio, Methylen, oder einen zweiwertigen Rest der Teilformel

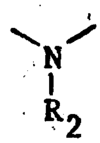

(Ia)

bedeutet, in der $R_2$ Wasserstoff oder Niederalkyl darstellt, sowie Salze von solchen Verbindungen, dadurch gekennzeichnet, dass man

a) einen reaktionsfähigen Diester eines Dimethanols der Formel

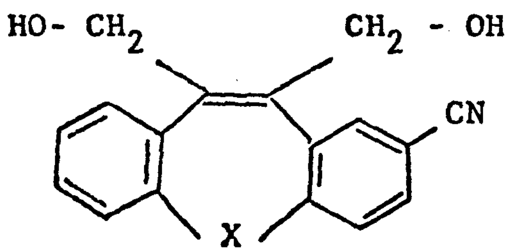

(II)

mit einer Verbindung der Formel

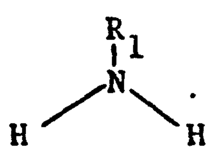

(III)

umsetzt, oder

b) eine Verbindung der Formel

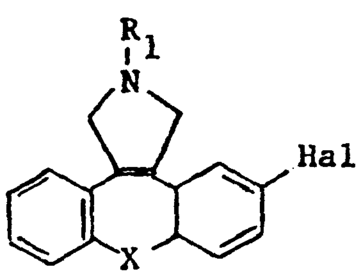

(IV)

in welcher Hal ein Halogenatom darstellt, mit einer Cyanidverbindung umsetzt, und, wenn erwünscht, eine verfahrensgemäss erhaltene Verbindung der Formel I in eine andere Verbindung der Formel I umwandelt, und/oder, wenn erwünscht, ein verfahrensgemäss erhaltenes Salz in die freie Verbindung oder in ein anderes Salz oder eine verfahrensgemäss erhaltene freie Verbindung in ein Salz umwandelt.

0007450

Europäisches Patentamt

# EUROPÄISCHER RECHERCHENBERICHT

Nummer der Anmeldung

EP 79 102 146.2

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | DE - A - 1 959 405 (GEIGY)<br>* Ansprüche 1, 4 *<br>-- | 13-16 |
| D | DE - A - 2 125 634 (CIBA-GEIGY)<br>* Ansprüche 1, 11 *<br>-- | 13-16 |
| | DE - A - 2 125 892 (CIBA-GEIGY)<br>* Ansprüche 1, 17 *<br>-- | 13-16 |
| | DE - A1 - 2 506 155 (CIBA-GEIGY)<br>* Ansprüche 1, 4 *<br>-- | 13-16 |
| A | DE - A1 - 2 723 209 (AKZO)<br>---- | |

KLASSIFIKATION DER ANMELDUNG (Int.Cl.3)

C 07 D 495/04
C 07 D 491/04
A 61 K 31/335
A 61 K 31/38
A 61 K 31/40
A 61 K 31/55//
(C 07 D 491/04, 313/00,209/00)
(C 07 D 495/04, 337/00,209/00)

RECHERCHIERTE SACHGEBIETE (Int. Cl.3)

A 61 K 31/335
A 61 K 31/38
A 61 K 31/40
A 61 K 31/55
C 07 D 209/94
C 07 D 487/04
C 07 D 491/04
C 07 D 495/04

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 05-11-1979 | FROELICH |